# EUROPEAN PATENT SPECIFICATION

(11) **EP 4 401 587 B1**
(45) Date of publication and mention of the grant of the patent: **02.12.2026**
(21) Application number: 21957172.6
(22) Date of filing: 18.09.2021
(51) Int. Cl.: A24F 40/46, A61M 15/06

(54) **CARTRIDGE FOR MICROVAPORIZER**
KARTUSCHE FÜR MIKROVERDAMPFER
CARTOUCHE POUR MICROVAPORISATEUR

(43) Date of publication of application: 24.07.2024
(73) Proprietor: Central Victory Limited HK, Admiralty, Hong Kong (HK)
(72) Inventor: XIE, Haojun, Baise, Guangxi 531499 (CN)
(74) Representative: Abel & Imray LLP
(86) International application number: PCT/CN2021/119417
(87) International publication number: WO 2023/039895

(56) References cited:
- WO-A1-2019/206985
- WO-A1-2020/025644
- WO-A1-2020/223875
- WO-A1-2020/223876
- CN-A- 107 455 800
- CN-A- 107 635 418
- CN-A- 108 136 141
- CN-A- 109 414 060
- CN-A- 109 549 253
- CN-A- 113 163 865
- CN-U- 206 079 043
- GB-A- 2 542 018
- KR-B1- 102 148 142
- US-A1- 2008 092 912
- US-A1- 2020 008 473

## Description

### FIELD

The technology relates to a cartridge for microvaporizer, and
particularly to a cartridge that includes an electrically heated element incorporated into the cartridge.

### BACKGROUND

Microvaporizers, also referred to as vaping devices, provide alternatives to cigarettes, cigars, pipes and other tobacco smoking devices. Vaping devices may be configured to provide the sensations associated with cigarette, cigar, or pipe smoking, but without delivering considerable quantities of incomplete combustion and pyrolysis products that result from the burning of tobacco. Microvaporizers may also be configured to deliver medicinal aerosols, such as asthma breathers.

Conventional microvaporizer heaters typically include a coiled heating wire is wrapped around a wick that draws a liquid infused with chemicals (such as nicotine) from a reservoir. Coiled heating wires heat the liquid in the wick, which may not all be vaporized. Thus, the coiled heating wires are inefficient in that the heat more liquid than is needed to create the aerosol. Further, coiled heating wires heat the outer surface of the wick to a greater extent than the interior of the wick and my not uniformly heat the surface of the wick. Thus, the coiled heating wire design may lead to inconsistent heating of the liquid which affects the size(s) of particles in the aerosol formed by heating the wick. The taste and user experience of inhaling the aerosol may be adversely affected by many variables such as inconsistent heating, surface area and aerosol particles of varying sizes.

In addition, the conventional coiled heating wire and wick heats the entire wick within the coil. Thus, there is only one operable heating zone. The conventional coiled heating wire is incapable of utilizing multi-zone heating, and must vary the magnitude of electrical power applied to the coil in order to regulate the temperature of the liquid flowing through the microvaporizer. The single-zone configuration has less control over the temperature of the liquid in the microvaporizer and allows for greater fluctuations in temperature, which in turn leads to greater fluctuations in particle size within the aerosol.

US2020/008473A1 (HAUNI MASCHINENBAU) discloses a vaporizer head for an inhaler, in particular for an electronic cigarette product, comprising a heating element-wick structure assembly consisting of an electrically operable heating element, which has at least one liquid-conducting through-channel and a porous and/or capillary wick structure arranged at an inlet side of the heating element, and a support plate for retaining the heating element-wick structure assembly. The vaporizer head has a liquid-conducting cannula having a preferably protruding piercing region.

WO2020/025644A1 (PHILIP MORRIS PRODUCTS SA [CH]) discloses a cartridge for an electronic vaping device comprising: an outer housing extending in a longitudinal direction; a reservoir configured to contain a pre-vapor formulation, the reservoir in the outer housing; at least two transfer pads at a reservoir end, the transfer pads including a plurality of fibers, each of the plurality of fibers being substantially parallel to the longitudinal direction; and a wick in contact with the transfer pads.

WO2019/206985A1 (PHILIP MORRIS PRODUCTS SA [CH]) discloses a vaporizer assembly for an electrically operated aerosol-generating device comprising: a generally planar, fluid permeable heating element having a first side and a second side opposite the first side and a liquid transport medium, the liquid transport medium having a first side in contact with the second side of the heating element and a second side opposite to the first side. The heating element extends over a first area of the first side of the liquid transport medium. A liquid supply conduit having a first end is in contact with the second side of the liquid transport medium and extends over only a second area of the second side of the liquid transport medium, wherein the second area is smaller than the first area. The liquid transport medium is arranged to transport liquid from the liquid supply conduit to the first area of the second side of the heating element. Having the liquid supply conduit extend over a relative small area of the liquid transport medium compared to the heating element has the advantage that only a small proportion of the heat generated by the heater is transferred to the liquid in the liquid supply conduit. This provides good heating efficiency for the vaporizer assembly.

### SUMMARY

The invention is defined by the appended claims.

Aspects of the cartridge and the associated microvaporizer described herein provide solutions to one or more problems or disadvantages associated with the prior art.

In a first aspect of the technology, a heater assembly may be configured to vaporize a liquid. The heater assembly may include a substrate plate and a heating element supported on the substrate plate.

In another aspect of the technology, a cartridge may include the heater assembly.

In yet another aspect of the technology, a microvaporizer may include a base and a cartridge with a heater assembly.

In yet another aspect of the technology, a cartridge is configured to be mounted to a microvaporizer device the cartridge includes a hollow main body and a reservoir located within the hollow main body. The reservoir is configured to contain a body of liquid. The cartridge also includes a heater located within the hollow main body and configured to transform the liquid in the reservoir to an aerosol and/or a vapor. A hydrophilic member is positioned between the reservoir and the heater. The hydrophilic member is configured to directly receive the liquid from the reservoir, and the heater is configured to heat the liquid after it has been received by the hydrophilic member. The cartridge also includes a vapor passage within the hollow main body configured to deliver the aerosol and/or vapor to a user interface wherein the reservoir is positioned between the user interface and the heater, and wherein an inlet end of the hydrophilic member abuts an outlet of the reservoir.

The vapor passage may terminate at a mouthpiece, and the mouthpiece may be integrally formed with the hollow main body.

The hydrophilic member may be configured to absorb the liquid from the reservoir, and the heater may be configured to heat the liquid absorbed by the hydrophilic member.

The hydrophilic member may be configured to draw the liquid from the reservoir by way of capillary action.

The heater may be configured to heat liquid on a surface of the hydrophilic member.

The hydrophilic member may be made of natural fibers such as cotton.

The hydrophilic member may comprise a hydrophilic coating.

The heater may be configured to vaporize liquid in the hydrophilic member.

The heater is a flat heater comprising a thin plate of electrically conductive material that may be thinner than the hydrophilic member.

The heater and the hydrophilic member may be positioned at a first end of the cartridge opposite a second end of the cartridge where the user interface is located.

The cartridge may lack moving parts.

In yet another aspect of the technology, a microvaporizer device is configured to deliver an aerosol to a user's airways and includes a base comprising a power supply and a cartridge as disclosed in any one of paragraphs discussed above.

The cartridge may be removably connected to the base.

The cartridge may be configured to be attached to the base by way of a snap-fit connection.

The cartridge and the base may be configured so that an audible signal is generated when the cartridge is secured to the base.

The cartridge may be configured to be attached to the base by way of an interference fit connection.

The microvaporizer may lack moving parts.

In yet another aspect of the technology, a cartridge is configured to be mounted to a microvaporizer device and includes a hollow main body, a user interface positioned within the hollow main body, a reservoir located within the hollow main body, a heater located within the hollow main body, and a vapor passage extending from the heater to the user interface. The heater is configured to transform the liquid in the reservoir to an aerosol and/or a vapor. The reservoir is positioned between the user interface and the heater. In addition, the liquid reservoir is configured to contain a body of liquid.

The vapor passage may be adjacent the reservoir.

The vapor passage and the reservoir may be separated from each other by an interior wall within the hollow main body, and the interior wall may extend from the user interface to the heater.

The hollow main body may be transparent.

The heater may be located at a first end of the hollow main body that is opposite a second end of the hollow main body where the user interface is located.

The heater is a flat heater comprising a thin plate of electrically conducting material.

The reservoir may have a larger volume than the vapor passage.

The cartridge may further include a hydrophilic member configured to draw liquid from the reservoir.

The heater may be configured to vaporize liquid on a surface of the hydrophilic member.

None of the components of the cartridge may have moving parts.

In yet another aspect of the technology, a microvaporizer device may be configured to deliver an aerosol to a user's airways and may include a base comprising a power supply and a cartridge as disclosed in any of the paragraphs above.

The cartridge may be removably connected to the base.

The cartridge may be configured to be attached to the base by way of a snap-fit connection.

The cartridge may be configured to be attached to the base by way of an interference fit connection.

The cartridge and the base may be configured so that an audible signal is generated when the cartridge is secured to the base.

The cartridge may be configured so that securing the cartridge to the base completes an electrical circuit that supplies electric power to the heater.

The microvaporizer may lack moving parts.

In yet another aspect of the technology, a cartridge is configured to be mounted to a microvaporizer device and includes a hollow main body, a reservoir located within the hollow main body, a flat heater located within the hollow main body, and a vapor passage within the hollow main body configured to deliver the aerosol and/or vapor to a user interface. The liquid reservoir is configured to contain a body of liquid. The flat heater is configured to transform the liquid in the reservoir to an aerosol and/or a vapor and comprises a thin plate of electrically conductive material.

The flat heater may be a two-stage heater.

The flat heater includes a first heating zone configured to generate a first magnitude of heat and a second zone configured to generate a second magnitude of heat that is greater than the first magnitude. The first zone may be located at a central region of the heater. The second zone may be located at a perimeter of the heater. The first zone may be located between two sections of the second zone. The second zone may be divided into two sections that are located at opposite sides of the heater.

The cartridge may further include a hydrophilic member configured to draw liquid from the reservoir.

The heater may be configured to vaporize liquid on a surface of the hydrophilic member.

In yet another aspect of the technology, a microvaporizer device is configured to deliver an aerosol to a user's airways and includes a base comprising a power supply and a cartridge as disclosed in any one of paragraphs above.

The cartridge may be removably connected to the base.

The cartridge may be configured to be attached to the base by way of a snap-fit connection.

The cartridge may be configured to be attached to the base by way of an interference fit connection.

The cartridge may be configured so that securing the cartridge to the base completes an electrical circuit that supplies electric power to the heater.

### BRIEF DESCRIPTION OF THE DRAWINGS

Fig. 1 shows an exemplary microvaporizer including a base and a cartridge.
Fig. l A shows a cross-section of the recess in the base of the microvaporizer.
Fig. 2 shows the cartridge of Fig. 1.
Fig. 3 shows a cross-sectional view of the cartridge and the base.
Fig. 4 shows an exemplary heater element of the cartridge.
Figs. 5-12 show alternative exemplary heater elements.

### DETAILED DESCRIPTION OF EXAMPLE NON-LIMITING EMBODIMENTS

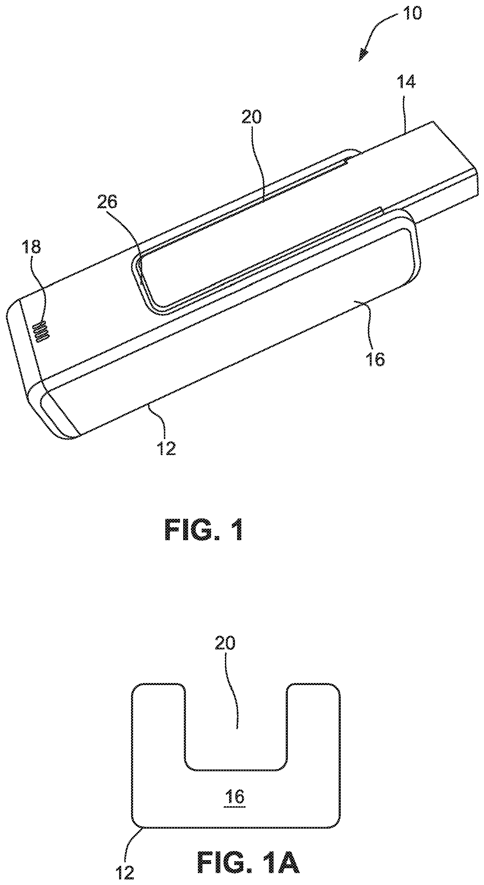

Fig. 1 shows an exemplary microvaporizer 10 for generating an aerosol for inhalation by a user. The microvaporizer 10 may be configured as a vaping device for delivery of a nicotine vapor to the mouth of a user. The microvaporizer 10 may also be configured to deliver a medicinal vapor, such as an aerosol infused with asthma drugs, to the mouth of a user. Moreover, the microvaporizer 10 may be configured for use to deliver other types of vapor (aerosols) to a user.

The microvaporizer 10 may include a base 12 and a cartridge 14. The base 12 may be a hollow handheld device with outer surfaces shaped to be easily held in one hand and carried in a user's pocket or purse. The outer surfaces may form a casing (housing) 16 that houses a power source such as a battery (not shown) and electronic circuits (not shown) that are configured to deliver electrical power from the power source to a heater in the cartridge 14.

The casing (or housing or hollow body) 16 of the base 12 may be transparent, translucent, or opaque (or one more parts of the casing 16 may be transparent while other parts are translucent or opaque). In addition, the casing 16 may include one or more vent openings 18. The vent openings 18 are shown in Fig. 1 as being an array of slits. However, the vent openings 18 may take any form that is capable of venting excess heat generated by the power source and/or electronic circuits contained within the casing 16.

It is contemplated that the base 12 may not house a motor or pump or any component with moving parts. Alternatively, the base 12 may include a motor and/or pump that interact with the cartridge 14 to move fluid contained within the cartridge 14.

The base 12 may include a recess 20 shaped complimentarily to the shape of the cartridge 14 so that the cartridge 14 may be received within the recess 20. In one example, the recess 20 may extend only part of the length of the base 12. In another example, the recess 20 may extend the full length of the base 12. Fig. 1 shows a configuration in which the recess 16 extends all the way to one end of the base 12 and only part of the way to the other end of the base 12. In this configuration, a side wall of the base 12 may have a cut out in a shape that is similar to part of the cross-sectional shape of the cartridge 14. For example, the cross-sectional shape of the cut out in the side wall of the base 12 may be a flattened U-shape (see Fig. 1A), a V-shape, a semicircular shape, or any other shape that can receive the cartridge 14. It is also contemplated that a length of the recess 20 may be sized so that a portion of the cartridge 14 extends beyond a side of the base 12 so that a part of the cartridge 14 is not supported by the base 12. The portion of the cartridge 14 that is not supported by the base 12 may be a user interface (e.g., a mouthpiece) 22.

The recess 20 and the cartridge 14 may be constructed so that the cartridge 14 is inserted into the recess 20 by sliding the cartridge 14 into the recess 20 from one end of the base 12 until a contact wall 24 of the cartridge 14 abuts a stop wall 26 of the recess 20. The stop wall 26 may include one or more electrical contacts configured to engage corresponding electrical contacts on the contact wall 24 of the cartridge 14. In this configuration, fully inserting the cartridge 14 into the recess 20 may automatically connect the cartridge 14 to the power source in the base 12. It is contemplated that other walls of the recess 20 may also include electrical contacts configured to engage corresponding electrical contacts on the cartridge 14.

The stop wall 26 of the base 12 may also comprise interlocking components that cooperate with corresponding interlocking components on the cartridge 14 to secure the cartridge 14 to the base 12. The interlocking components may be part of a "snap-fit" connection with projections that are resiliently inserted into recesses or openings. The projections and recesses or openings may be located in the contact wall 24 of the cartridge and the stop wall 26 of the base 12. It is contemplated that other walls of the base 12 and the cartridge 14 may include the projections and recesses or openings.

It is further contemplated that the cartridge 14 and the base 12 may be secured to each other by other means. For example, the shape and size of the recess 20 may allow for an interference or friction fit of the cartridge 14 within the recess 20. In addition, the cartridge 14 may generate a signal upon being secured to the base 12 to indicate to the user that the cartridge 14 is secured to the base 12. The signal may be, for example, an audible noise such as a click, pop, or snap. The signal may also be a light indicator that illuminates upon an electrical circuit being complete when the cartridge 14 is secured to the base 12.

As illustrated in Figs. 1 to 4, the cartridge 14 may include the user interface 22, a casing (housing or hollow main body) 28 that houses the internal components of the cartridge 14, a reservoir 30, a heater assembly 32, and a vapor path 34.

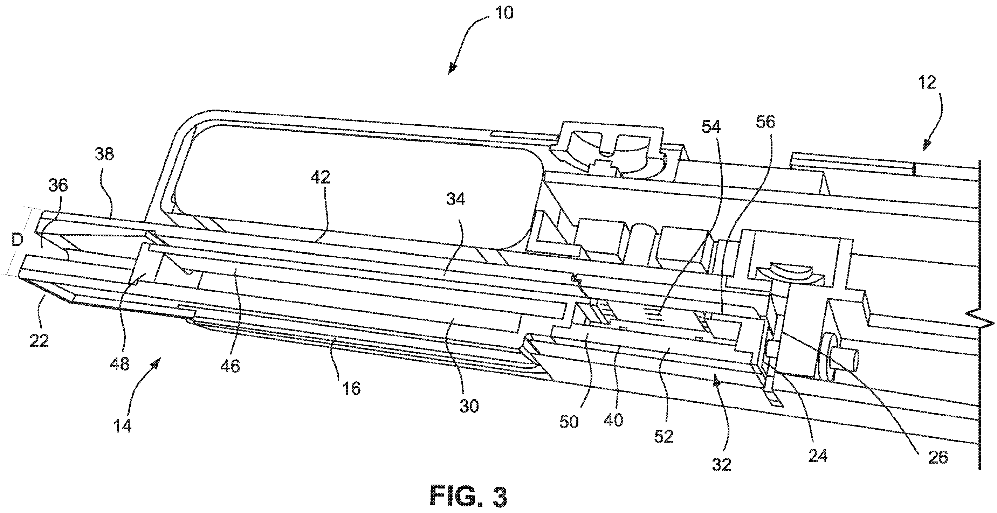

The user interface 22 may be part of the casing 28 or integrally formed with the casing 28. Alternatively, the user interface 22 may be separately formed from the casing 28 and may be permanently attached to the casing 28 or removably attached to the casing 28. As can be seen in Fig. 3, the interface 22 may have a hollow interior that decreases in volume toward a discharge opening 36 that discharges vapor to the user's airways. The change in volume may be achieved by gradually increasing the wall thickness of the user interface 22 toward the discharge opening 36. Alternatively, the volume of the hollow interior may be consistent throughout the user interface 22.

The casing 28 may be transparent, translucent, or opaque (or one more parts of the casing 16 may be transparent while other parts are translucent or opaque). It is contemplated that the casing 28 may be color coded so that the color of the casing 16 may indicate the type of fluid contained within the cartridge (e.g., medicinal drug, nicotine) and/or a flavor of the fluid contained within the cartridge.

The casing 28 may be substantially rectangular (i.e., the casing 28 may be mostly rectangular with some minor non-rectangular features such as recesses and/or projections and filleted or rounded corners). In addition, the casing 28 may have a user interface end 38, a heater end 40 opposite the user interface end 38, and a mid-section 42 between the user interface end 38 and the heater end 40. It is contemplated that a depth D of the casing 28 may be less at the heater end 40 than at the mid-section 42 and/or the user interface end 38 (e.g., the heater end 40 may be recessed relative to the mid-section 42 and/or the user interface end 38). Alternatively, the depth D of the casing 28 may be consistent throughout the casing 28.

For a configuration in which the user interface 22 is part of the casing 28, the user interface end 38 of the casing 28 may terminate at the discharge opening 36. However, if the user interface 22 is separately formed from the casing 28, the user interface end 38 of the casing 28 may include a connection structure to attach the user interface 22 to the casing 28. For example, the user interface end 38 may have a universal connection so that more than one size or type of user interface 22 may be attached to the casing 28.

The heater end 40 of the casing 28 may be located at the end of the cartridge 14 that is opposite to the user interface 22 and may house the heater assembly 32. The heater end 40 may also include the contact wall 24 of the cartridge 14. One or more electrical contacts 44 may be located on the contact wall 24 and may be positioned to be brought into contact with corresponding electrical contacts on the base 12. It is contemplated that other surfaces of the casing 28 may include electrical contacts.

The mid-section 42 of the casing 28 may house the reservoir 30 and the vapor path 34. It is contemplated that the heater end 40 may also house part of the vapor path 34. The reservoir 30 and the vapor path 34 may be positioned adjacent to each other (side by side). An interior wall 46 may extend the length of the mid-section 42 and may divide the mid-section 42 into the reservoir 30 and the vapor path 34. In other words, the reservoir 30 and the vapor path 34 may be separated by the interior wall 46. In addition, it is contemplated that the cross-sectional area of the reservoir 30 may be greater than the cross-sectional area of the vapor path 34.

The reservoir 30 may be positioned between the user interface 22 and the heater assembly 40 (e.g., the reservoir 30 may extend from the user interface 22 to the heater assembly 40). In addition, the reservoir 30 may be separated from the user interface 22 by an interior wall 48 that extends the width and depth of the reservoir 30. The interior wall 48 may prevent any fluid in the reservoir 30 from entering the user interface 22 before being vaporized. An opening (outlet) 50 at an opposite end of the reservoir 30 may allow the fluid in the reservoir 30 to enter the heater end 40 of the casing 28, where the fluid can be vaporized by the heater assembly 32. It is contemplated that the reservoir 30 may be located at the portion of the cartridge 14 having the greatest depth D in order to maximize the amount of fluid the cartridge can hold. For disposable versions of the cartridge 14, the reservoir may be prefilled with the fluid during manufacturing. Alternatively, for reusable versions of the cartridge 14, the casing 28 may include a refill opening (not shown) that may allow the reservoir 30 to be refilled with the fluid. The fluid may be a liquid infused with chemicals (such as nicotine).

The heater assembly 32 may be located on an opposite end of the cartridge 14 from the user interface 22. This location may allow the heater assembly 32 to be directly connected to the electrical circuitry in the base 12, thereby reducing the amount electrical circuitry within the cartridge 14. In addition, the heater assembly 32 may be positioned to receive the fluid from the reservoir 30, vaporize the fluid, and then discharge the fluid to the vapor path 34. The heater assembly 32 may include a hydrophilic member 52, a heater element 54, and a substrate 56 that may support the heater element 54.

The hydrophilic member 52 may be positioned to directly receive the fluid from the reservoir 30. For example, the hydrophilic member 52 may abut the outlet 50 of the reservoir 30 so that the hydrophilic member 52 may draw or absorb the fluid from the reservoir 30. The absorbing or drawing action of the hydrophilic member 52 may allow the cartridge to function without the need of a motor or pump to circulate the fluid in the reservoir. In other words, similar to the base 12, the cartridge may lack a motor or pump or any component with moving parts. Alternatively, the cartridge 14 may include a motor and/or pump that moves fluid contained within the cartridge 14.

The absorbing or drawing action of the hydrophilic member 52 may also automatically regulate the flow rate of the fluid to the heater assembly 32. In particular, the retention capacity of the hydrophilic member 52 may limit the amount of fluid that enters the heater assembly 32. Once the fluid in the hydrophilic member 52 is at capacity, the hydrophilic member 52 may stop absorbing or drawing the fluid from the reservoir 30 until the amount of fluid in the hydrophilic member 52 falls below the retention capacity of the hydrophilic member 52. It is contemplated that the hydrophilic member 52 can be made of any type of hydrophilic material such as, for example, cotton or other natural or artificial hydrophilic fiber. It is also contemplated that the hydrophilic member may be a component with a hydrophilic coating.

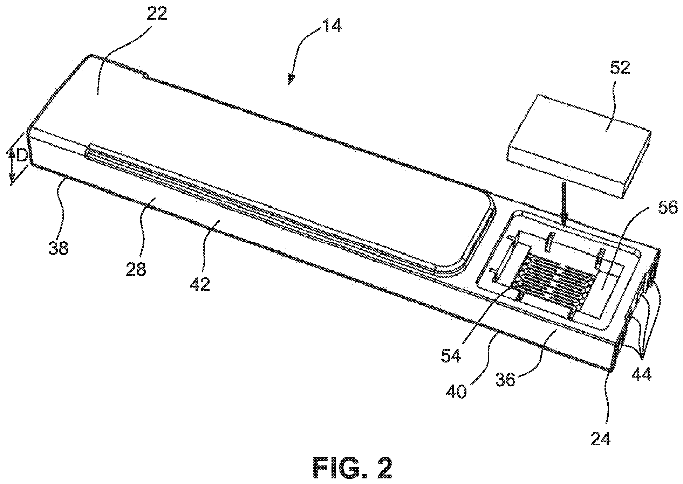

As shown in Figs. 2-4, the heater element 54 may be positioned between the hydrophilic member 52 and the vapor path 34. The hydrophilic member 52 may be in contact with a receiving face 58 of the heater element 54. Alternatively, there may be a gap between the hydrophilic member 52 and the receiving face 58 of the heater element 54. However, in the spaced apart configuration, the gap may be small enough that fluid in the hydrophilic member 52 may still be vaporized by the heater element 54.

It should be understood that the heater element 54 may generate the heat necessary for heating and vaporizing the fluid (or transforming the fluid into an aerosol to be delivered to the user's airways). It is contemplated that the configuration or structure of the heater element 54 (in conjunction with the structure of the hydrophilic member 52) may induce the fluid to flow through the cartridge 14 by way of, for example, capillary action without the need for a pump. In particular, the hydrophilic member 52 may draw fluid from the reservoir by absorption and/or capillary action. The heater element 54 may then vaporize the liquid in the hydrophilic member 52 (and/or on a surface of the hydrophilic member 52) and/or or transform the liquid into an aerosol form, thereby causing the fluid to flow from the hydrophilic member 52 through the heater element 54 to the vapor passage 34. The hydrophilic member 52 may then be free to draw more liquid from the reservoir 30 to replace the liquid lost to vaporization and/or the transformation to aerosol.

The heater element 54 is in the form of a flat heater that may be thinner than the hydrophilic member 52. In addition, the heating element 54 is made from electrically conducting material such as, for example, a metal or semiconductor. Different parts of the heating element 54 may be made of different types of material with different electrically conducting characteristics. The overall shape and the individual components of the heating element 54 may be carved, cut, punched, or etched from a blank. The heater element 54 may include a supporting frame 60, one or more electrical contacts 62, and a vaporizing section 64.

The supporting frame 60 may form a perimeter of the heater element 54 and may be the part of the heater element 54 that supports the electrical contacts 44 and the vaporizing section 64. The supporting frame 60 may also include connectors 66 that secure the heater element 54 to the interior of the casing 28. The connectors 66 may be in the form of clips positioned along the edge of the supporting frame 60 that engage corresponding sockets or openings in the interior of the casing 28 (or an interior wall or substrate within the casing 28). It is also contemplated that the supporting frame 60 may rest on and be secured to a shelf or ledge within the casing 28. The supporting frame 60 may be secured to the substrate 56 by other means such as adhesives, bonding, or other types of mechanical fasteners.

The one or more electrical contacts 44 may be positioned on an outer side of the casing 28 (at the contact wall 24 of the cartridge 14) so that the one or more electrical contacts 44 may directly engage electrical contacts on the base 12 when the cartridge 14 is secured to the base 12. The one or more electrical contacts 44 may be connected to the supporting frame 60 so that electricity generated by the electric power source in the base 12 can be directed from the one or more electrical contacts 44, through the supporting frame 60, and subsequently to the vaporizing section 64.

The vaporizing sections 64 may be located in a central portion of the heater element 54. In addition, the majority of the heat generated by the heater element 54 may be generated in the vaporizing section 64. The vaporizing section 64 may include a plurality of strips of material that are at least partially separated from each other by gaps in the electrically conducting material. The gaps in the electrically conducting material may also at least partially separate the strips of material in the vaporizing section 64 from the supporting frame 60.

In one exemplary configuration, the strips of material of the vaporizing section 64 may be in the form of an array of loops 68 and a plurality of cantilevered extensions 70 at the ends of each loop 68. Each loop 68 may include a mid-section 72 and two opposing end-sections 74. Adjacent loops 68 may be connected to each other at their mid-sections 72. In addition, the outermost loops 68 may be connected to the supporting frame 60 at their mid-sections 72. Also each cantilevered extension 70 may extend from a respective end-section 74 so that there are an equal number of end-sections 74 and cantilevered extensions 70.

The vaporizing section 64 may generate heat by way of resistance heating. The heating element 54 utilizes multi-stage heating wherein, in the present embodiment, the fluid received from the hydrophilic member 52 is exposed to increasing levels of heat from the loops 68 to the cantilevered extensions 70. Given that the amount of heat generated in a resistance heater depends on the magnitude of the electrical resistance in the material to which electricity is applied, for multi-stage heating, the loops 68 may have a different electrical resistance value than the cantilevered extensions 70. In particular, the cantilevered extension 70 may have a greater resistance than the loops 68.

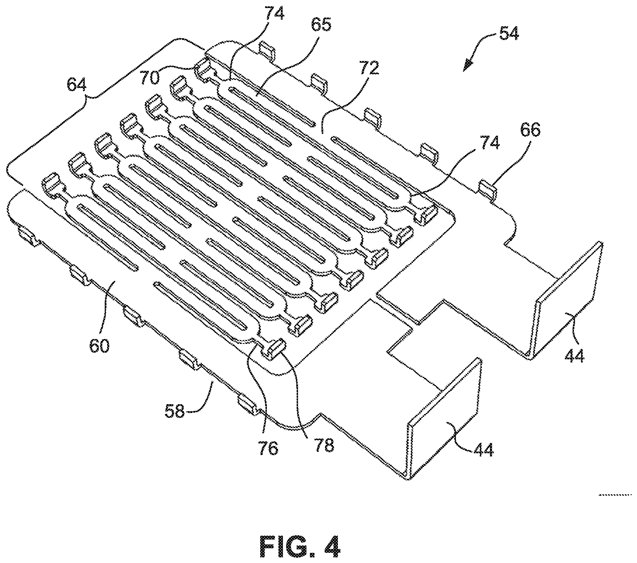

One way of achieving different electrical resistances is to vary the width (or cross-sectional shape) of the electrically conducting material. For example, as shown in Fig. 4, the electrically conducting material forming the loops 68 may be wider (or have a larger cross-section) than at least part of the electrically conducting material of the cantilevered extensions 70. Accordingly, the loops 68 may have a smaller electrical resistance and may generate a smaller amount of heat than at least part of the cantilevered extensions 70.

It is contemplated that the material in each loop 68 may have a consistent width W at the mid-section 72 and at the end-sections 74. In addition, each cantilevered extension 70 may include a necked region 76 (adjacent to a corresponding end-section 74) at which the width W of the electrically conducting material is reduced or narrowed. The width W of the cantilevered extension 70 may be increased at a terminal end 78 that is adjacent to the necked region 76 so that the narrowest portion of the electrically conducting material is at the necked regions 76. It is further contemplated that the width W of the terminal end 78 may be less than, the same, or greater than the width W of the loops 68. Accordingly, the part of the vaporizing section 64 producing the greatest heat may be the necked regions 76 of the cantilevered extensions 70.

Multi-stage heating may allow for better control over the temperatures applied to the fluid received from the hydrophilic member 52. Since different temperatures generate different particle sizes when forming an aerosol, better control over the temperature may allow for better control over the size of the particles formed in the aerosol generated by vaporizing the fluid in the heating element 54. Different particle sizes are desired depending on the use of the microvaporizing device 10. For example, nicotine absorption requires smaller particle sizes for absorption in the user's lungs, while larger particle sizes improve the taste of the aerosol.

In addition to multi-stage heating between the loops 68 and the cantilevered extensions 70, the vaporizing section 64 also has multi-zone heating which, in the present embodiment, operates across different loops 68 and/or cantilevered extensions 70. In particular, the different loops 68 may be segmented into separately actuated groups. Accordingly, not only can the amount of heating be controlled by staging the amount of heat applied between the loops 68 and the cantilevered extensions 70, the amount of heat can also be controlled by actuating one, some or all of the separately actuated groups of loops 68 and/or cantilevered extensions 70. Each group of loops 68 and/or cantilevered extensions 70 may be associated with a particular heating temperature range and/or electrical resistance range. Also, the electrical current applied to each group of loops 68 and/or cantilevered extensions 70 may be selected to achieve a desired heating of the fluid in the respective sections of the vaporizing section 64.

The multi-zone heating across the different loops 68 and /or cantilevered extensions 70 may allow for the controlled generation of different sized particles within a common aerosol. As discussed above, nicotine absorption requires smaller particle sizes, while larger particle sizes improve the taste of the aerosol. Multi-zone heating across the different loops 68 and /or cantilevered extensions 70 may generate more than one particle size, thereby addressing the multiple particle size needs for a nicotine infused aerosol.

Multi-zone heating may also increase the efficiency of the heating element 54 by tailoring the amount of heating to the user's demand. For example, if the flow of aerosol inhaled by the user is low, only one or two groups of loops 68 and /or cantilevered extensions 70 may be activated to generate heat. If the user inhales more aerosol, more groups of loops 68 and /or cantilevered extensions 70 may be activated to generate more heat. Thus, utilizing multi-zone heating across different loops 68 and /or cantilevered extensions 70 can reduce the average amount of electrical power drawn by the heating element 54 by only utilizing the number of loops 68 and /or cantilevered extensions 70 required by the user's demand.

It is contemplated that in some configurations, all of the vaporization may be performed only by the cantilevered extensions 70. In this configuration, cantilevered extensions 70 on opposing sides of the heater element 54 may increase the vaporizing area and the amount of energy provided by the heater element 54 for vaporizing the liquid from the hydrophilic member 52 (as opposed to vaporizing only on one side of the heater element 54). It is contemplated that the loops 68 may provide intermediate heating that does not vaporize the liquid fluid but may instead raise the temperature of the liquid so that by the time the liquid fluid reaches the cantilevered extensions 70, the amount of energy needed to vaporize the liquid is less than what would otherwise be needed if the liquid was not preheated by the loops 68.

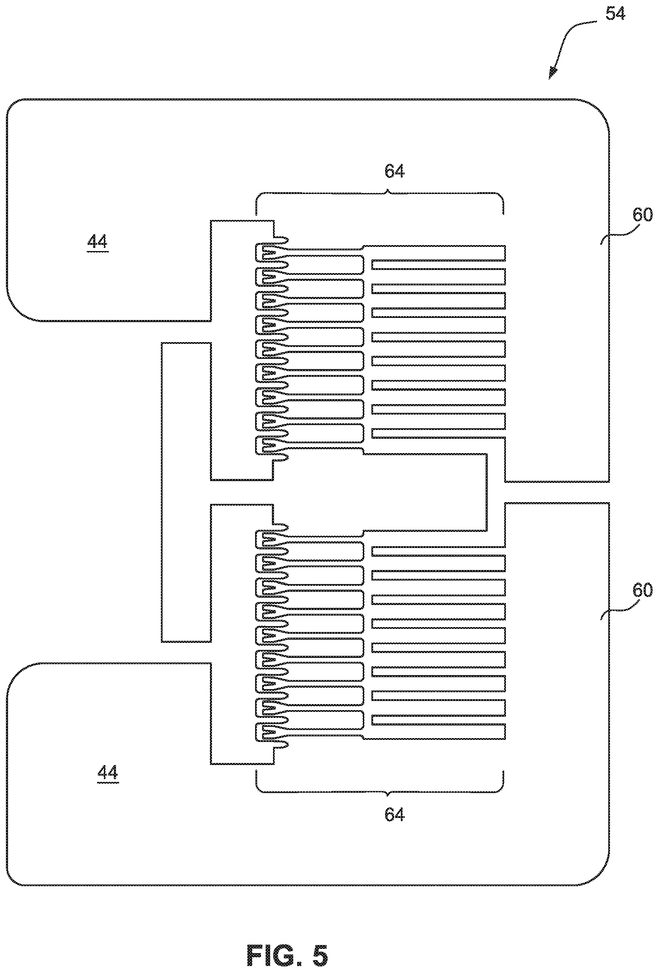

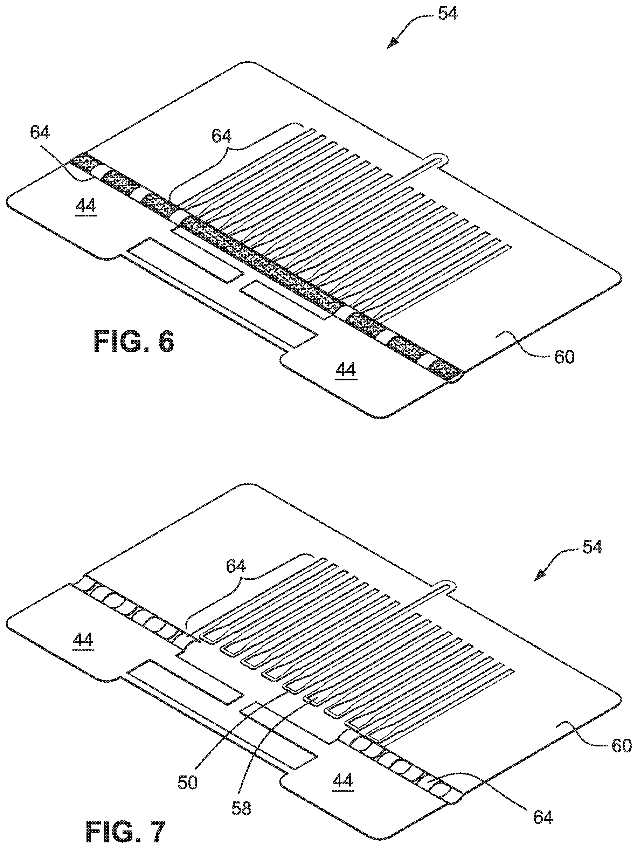

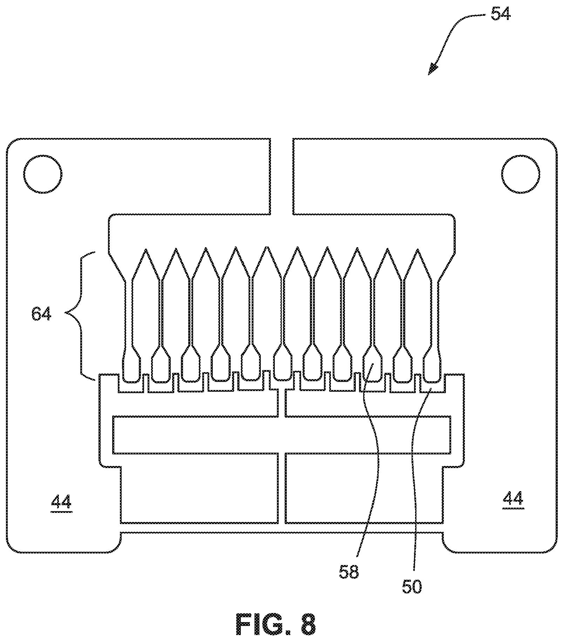

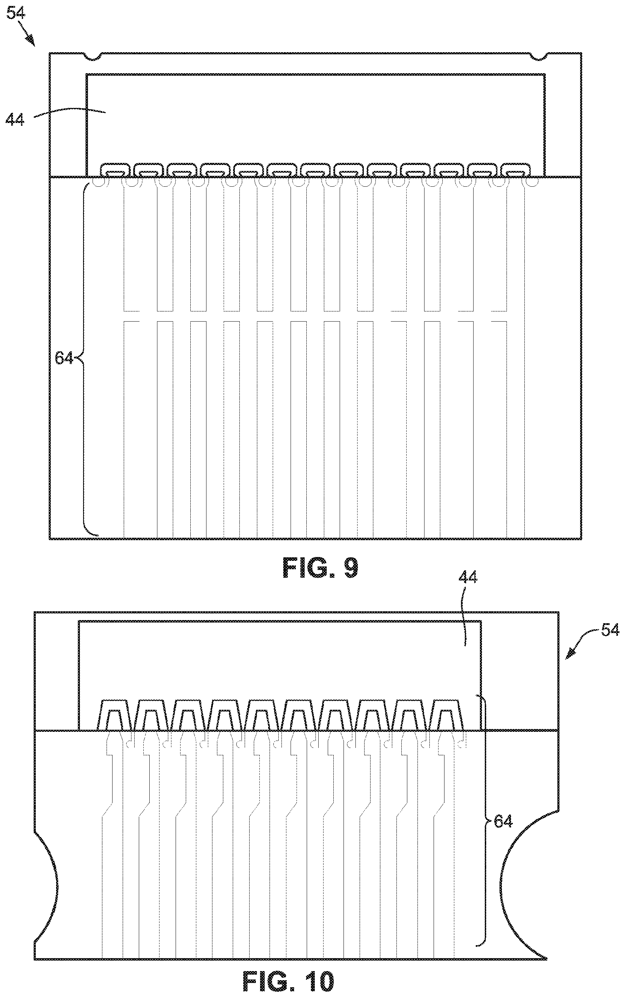

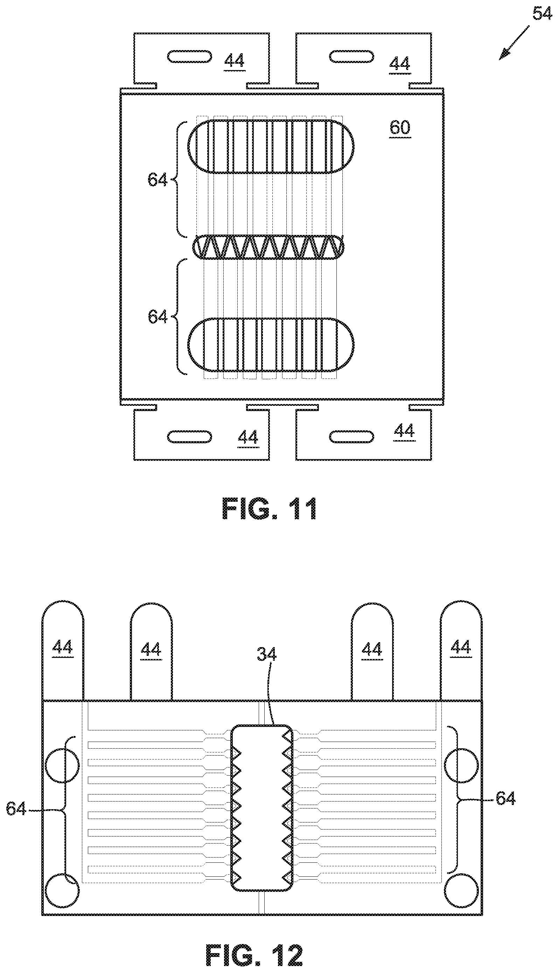

Fig. 4 illustrates one configuration of an exemplary flat heater usable with the cartridge 14. Figs. 5-12 illustrate other flat heater configurations.

Once vaporized (either over the entirety of the heater element 54 or only at the cantilevered extensions 70), the vapor may flow into the vapor path 34 by way of an open space adjacent to the heater element 54 on an opposite side of the heater element 54 from the hydrophilic member 52.

Once the heater element 54 vaporizes the fluid on the hydrophilic member 52 or transforms the liquid to an aerosol, the vapor and/or aerosol may be drawn through the vapor path 34 due to a pressure differential. For example, a patient inhaling through the user interface 22 may reduce the air pressure at the user interface 22, thereby creating a pressure differential across the vapor path 34 between the user interface 22 and the heater assembly 32. This may cause the vapor and/or aerosol to move from the higher pressure area adjacent the heater assembly 32 to the lower pressure area at the user interface 22. It is contemplated that the buildup of vapor and/or aerosol at the heater assembly end of the vapor path 34 may also create a pressure difference between the heater assembly end of the vapor path 34 and ambient pressure outside the cartridge. This pressure difference may also cause the vapor and/or aerosol to flow from the heater assembly end of the vapor path 34 to the user interface 22.

## Claims

1. A cartridge (14) configured to be mounted to a microvaporizer device (10), the cartridge (14) comprising:
a hollow main body (28);
a reservoir (30) located within the hollow main body (28), the reservoir (30) being configured to contain a body of liquid;
a flat heater (54) comprising a thin plate of electrically conductive material, located within the hollow main body (28) and configured to transform the liquid in the reservoir (30) to an aerosol and/or a vapor;
a hydrophilic member (52) positioned between the reservoir (30) and the flat heater (54), the hydrophilic member (52) being configured to directly receive the liquid from the reservoir (30) and the flat heater (54) being configured to heat the liquid after it has been received by the hydrophilic member (52); and
a vapor passage (34) within the hollow main body (28) configured to deliver the aerosol and/or vapor to a user interface (22),
wherein the reservoir (30) is positioned between the user interface (22) and the flat heater (54), and
wherein an inlet end of the hydrophilic member abuts an outlet of the reservoir;
**characterised in that** the flat heater (54) is a multi-stage flat heater (54) comprising a first heating zone configured to generate a first magnitude of heat and a second heating zone configured to generate a second magnitude of heat.

2. The cartridge (14) of claim 1, wherein the vapor passage (34) terminates at the user interface (22).

3. The cartridge (14) of any one of claims 1 to 2, wherein the user interface (22) is integrally formed with the hollow main body (28).

4. The cartridge (14) of any one of claims 1 to 3, wherein the hydrophilic member (52) is configured to absorb the liquid from the reservoir (30).

5. The cartridge (14) of any one of claims 1 to 4, wherein the flat heater (54) is configured to heat the liquid absorbed by the hydrophilic member (52).

6. The cartridge (14) of any one of clams 1 to 5, wherein the hydrophilic member (52) is configured to draw the liquid from the reservoir (30) by way of capillary action.

7. The cartridge (14) of any one of claims 1 to 6, wherein the flat heater (54) is configured to heat liquid on a surface of the hydrophilic member (52).

8. The cartridge (14) of any one of claims 1 to 7, wherein the hydrophilic member (52) is made of natural fibers.

9. The cartridge (14) of any one of claims 1 to 8, wherein the hydrophilic member (52) is made of cotton.

10. The cartridge (14) of any one of claims 1 to 9, wherein the hydrophilic member (52) comprises a hydrophilic coating.

11. The cartridge (14) of any one of claims 1 to 10, wherein the flat heater (54) is configured to vaporize liquid in the hydrophilic member (52).

12. The cartridge (14) of any one of claims 1 to 11, wherein the thin plate of electrically conducting material is thinner than the hydrophilic member (52).

13. The cartridge (14) of any one of claims 1 to 12, wherein the flat heater (54) and the hydrophilic member (52) are positioned at a first end of the cartridge (14) opposite a second end of the cartridge (14) where the user interface (22) is located.

14. The cartridge (14) of any one of claims 1 to 13, wherein the cartridge (14) does not have moving parts.

## Patentansprüche

1. Kartusche (14), die dazu konfiguriert ist, an einer Mikrovaporisatorvorrichtung (10) montiert zu werden, wobei die Kartusche (14)
einen hohlen Hauptkörper (28),
ein Reservoir (30), das sich innerhalb des hohlen Hauptkörpers (28) befindet, bei der das Reservoir dazu konfiguriert ist, eine Flüssigkeitsmenge aufzunehmen,
eine flache Heizvorrichtung (54) mit einer dünnen Platte aus elektrisch leitendem Material, die sich innerhalb des hohlen Hauptkörpers (28) befindet und dazu konfiguriert ist, die Flüssigkeit in dem Reservoir (30) in ein Aerosol und/oder einen Dampf umzuwandeln,
ein hydrophiles Bauteil (52), das zwischen dem Reservoir (30) und der flachen Heizvorrichtung (54) positioniert ist, bei der das hydrophile Bauteil (52) dazu konfiguriert ist, die Flüssigkeit direkt von dem Reservoir (30) zu empfangen, und die flache Heizvorrichtung (54) dazu konfiguriert ist, die Flüssigkeit zu erhitzen, nachdem sie durch das hydrophile Bauteil (52) aufgenommen wurde, und
eine Dampfpassage (34) innerhalb des hohlen Hauptkörpers (28) aufweist, die dazu konfiguriert ist, das Aerosol und/oder den Dampf zu einer Benutzerschnittstelle (22) zu liefern,
bei der das Reservoir (30) zwischen der Benutzerschnittstelle (22) und der flachen Heizvorrichtung (54) positioniert ist, und
bei der ein Einlassende des hydrophilen Bauteils an einen Auslass des Reservoirs grenzt,
**dadurch gekennzeichnet, dass** die flache Heizvorrichtung (54) eine mehrstufige flache Heizvorrichtung (54) ist, die eine erste Heizzone, die dazu konfiguriert ist, eine erste Wärmemenge zu erzeugen, und eine zweite Heizzone aufweist, die dazu konfiguriert ist, eine zweite Wärmemenge zu erzeugen.

2. Kartusche (14) nach Anspruch 1, bei der die Dampfpassage (34) an der Benutzerschnittstelle (22) endet.

3. Kartusche (14) nach einem der Ansprüche 1 bis 2, bei der die Benutzerschnittstelle (22) integral mit dem hohlen Hauptkörper (28) ausgebildet ist.

4. Kartusche (14) nach einem der Ansprüche 1 bis 3, bei der das hydrophile Bauteil (52) dazu konfiguriert ist, die Flüssigkeit aus dem Reservoir (30) zu absorbieren.

5. Kartusche (14) nach einem der Ansprüche 1 bis 4, bei der die flache Heizvorrichtung (54) dazu konfiguriert ist, die durch das hydrophile Bauteil (52) absorbierte Flüssigkeit zu erhitzen.

6. Kartusche (14) nach einem der Ansprüche 1 bis 5, bei der das hydrophile Bauteil (52) dazu konfiguriert ist, die Flüssigkeit mittels Kapillarwirkung aus dem Reservoir (30) anzusaugen.

7. Kartusche (14) nach einem der Ansprüche 1 bis 6, bei der die flache Heizvorrichtung (54) dazu konfiguriert ist, Flüssigkeit auf einer Oberfläche des hydrophilen Bauteils (52) zu erwärmen.

8. Kartusche (14) nach einem der Ansprüche 1 bis 7, bei der das hydrophile Bauteil (52) aus Naturfasern hergestellt ist.

9. Kartusche (14) nach einem der Ansprüche 1 bis 8, bei der das hydrophile Bauteil (52) aus Baumwolle hergestellt ist.

10. Kartusche (14) nach einem der Ansprüche 1 bis 9, bei der das hydrophile Bauteil (52) eine hydrophile Beschichtung aufweist.

11. Kartusche (14) nach einem der Ansprüche 1 bis 10, bei der die flache Heizvorrichtung (54) dazu konfiguriert ist, Flüssigkeit in dem hydrophilen Bauteil (52) zu vaporisieren.

12. Kartusche (14) nach einem der Ansprüche 1 bis 11, bei der die dünne Platte aus elektrisch leitendem Material dünner als das hydrophile Bauteil (52) ist.

13. Kartusche (14) nach einem der Ansprüche 1 bis 12, bei der die flache Heizvorrichtung (54) und das hydrophile Bauteil (52) an einem ersten Ende der Kartusche (14) entgegengesetzt zu einem zweiten Ende der Kartusche (14) positioniert sind, an welchem sich die Benutzerschnittstelle (22) befindet.

14. Kartusche (14) nach einem der Ansprüche 1 bis 13, wobei die Kartusche (14) keine beweglichen Teile aufweist.

## Revendications

1. Cartouche (14) configurée pour être montée sur un dispositif de microvaporisateur (10), la cartouche (14) comprenant :
un corps principal creux (28) ;
un réservoir (30) situé à l'intérieur du corps principal creux (28), le réservoir (30) étant configuré pour contenir un corps de liquide ;
un élément chauffant plat (54) comprenant une plaque fine constituée de matériau électriquement conducteur, situé à l'intérieur du corps principal creux (28) et configuré pour transformer le liquide dans le réservoir (30) en un aérosol et/ou une vapeur ;
un élément hydrophile (52) positionné entre le réservoir (30) et l'élément chauffant plat (54), l'élément hydrophile (52) étant configuré pour recevoir directement le liquide provenant du réservoir (30) et l'élément chauffant plat (54) étant configuré pour chauffer le liquide après qu'il a été reçu par l'élément hydrophile (52) ; et
un passage de vapeur (34) à l'intérieur du corps principal creux (28) configuré pour acheminer l'aérosol et/ou la vapeur vers une interface utilisateur (22),
dans laquelle le réservoir (30) est positionné entre l'interface utilisateur (22) et l'élément chauffant plat (54), et
dans laquelle une extrémité d'entrée de l'élément hydrophile vient buter contre une sortie du réservoir ;
**caractérisée en ce que** l'élément chauffant plat (54) est un élément chauffant plat (54) à multiples étages comprenant une première zone de chauffage configurée pour générer une première grandeur de chaleur et une seconde zone de chauffage configurée pour générer une seconde grandeur de chaleur.

2. Cartouche (14) selon la revendication 1, dans laquelle le passage de vapeur (34) se termine au niveau de l'interface utilisateur (22).

3. Cartouche (14) selon l'une quelconque des revendications 1 à 2, dans laquelle l'interface utilisateur (22) est formée d'un seul tenant avec le corps principal creux (28).

4. Cartouche (14) selon l'une quelconque des revendications 1 à 3, dans laquelle l'élément hydrophile (52) est configuré pour absorber le liquide depuis le réservoir (30).

5. Cartouche (14) selon l'une quelconque des revendications 1 à 4, dans laquelle l'élément chauffant plat (54) est configuré pour chauffer le liquide absorbé par l'élément hydrophile (52).

6. Cartouche (14) selon l'une quelconque des revendications 1 à 5, dans laquelle l'élément hydrophile (52) est configuré pour aspirer le liquide depuis le réservoir (30) par capillarité.

7. Cartouche (14) selon l'une quelconque des revendications 1 à 6, dans laquelle l'élément chauffant plat (54) est configuré pour chauffer du liquide sur une surface de l'élément hydrophile (52).

8. Cartouche (14) selon l'une quelconque des revendications 1 à 7, dans laquelle l'élément hydrophile (52) est constitué de fibres naturelles.

9. Cartouche (14) selon l'une quelconque des revendications 1 à 8, dans laquelle l'élément hydrophile (52) est constitué de coton.

10. Cartouche (14) selon l'une quelconque des revendications 1 à 9, dans laquelle l'élément hydrophile (52) comprend un revêtement hydrophile.

11. Cartouche (14) selon l'une quelconque des revendications 1 à 10, dans laquelle l'élément chauffant plat (54) est configuré pour vaporiser du liquide dans l'élément hydrophile (52).

12. Cartouche (14) selon l'une quelconque des revendications 1 à 11, dans laquelle la fine plaque constituée de matériau électriquement conducteur est plus fine que l'élément hydrophile (52).

13. Cartouche (14) selon l'une quelconque des revendications 1 à 12, dans laquelle l'élément chauffant plat (54) et l'élément hydrophile (52) sont positionnés à une première extrémité de la cartouche (14) opposée à une seconde extrémité de la cartouche (14) où est située l'interface utilisateur (22).

14. Cartouche (14) selon l'une quelconque des revendications 1 à 13, dans laquelle la cartouche (14) ne comporte pas de pièces mobiles.
